(19) Europäisches Patentamt
European Patent Office
Office européen des brevets

(11) **EP 2 044 886 A1**

(12) **EUROPEAN PATENT APPLICATION**

(43) Date of publication:
**08.04.2009 Bulletin 2009/15**

(51) Int Cl.:
*A61B 8/00* (2006.01)    *G10K 11/34* (2006.01)
*G01N 29/44* (2006.01)    *G01H 5/00* (2006.01)
*G01S 7/52* (2006.01)

(21) Application number: **08017303.2**

(22) Date of filing: **01.10.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **03.10.2007 JP 2007259919**
**19.09.2008 JP 2008241174**

(71) Applicant: **Fujifilm Corporation**
**Tokyo 106-8620 (JP)**

(72) Inventor: **Katsuyama, Kimito**
**Kanagawa (JP)**

(74) Representative: **Klunker . Schmitt-Nilson . Hirsch**
**Destouchesstrasse 68**
**80796 München (DE)**

(54) **Ultrasonic diagnosis method and apparatus**

(57)    The object of the present invention is achieved by providing an ultrasonic diagnosis apparatus (1) having an ultrasonic probe (10) in which a plurality of elements for transmitting an ultrasonic wave to a subject and, by receiving an ultrasonic signal reflected from the subject, outputting the received signal are arrayed; a device (12, 14) which changes an assumed sonic speed set in advance relative to the actual sonic speed of the ultrasonic wave to be transmitted to the subject; and an optimum sonic speed judgment device (20) which judges a micro-structure by an RF signal obtained from the received signal by changing the assumed sonic speed and performing focusing with a delay based on the assumed sonic speed, and judges an optimum sonic speed, which is the ultrasonic speed of the subject, from phase information about the RF signal judged to be the micro-structure.

FIG.12

**Description**

BACKGROUND OF THE INVENTION

Field of the Invention

The present invention relates to an ultrasonic diagnosis method and apparatus, and in particular to an ultrasonic diagnosis method and apparatus which takes and displays a tomographic image of a subject using an ultrasonic wave.

Description of the Related Art

It has been conventionally performed to acquire a tomographic image of a subject using an ultrasonic wave to use it for medical diagnosis. In an ultrasonic diagnosis apparatus, a tomographic image of a subject is acquired by giving delay time distribution to a plurality of received signals from a plurality of arrayed probe elements to form an ultrasonic beam having directivity toward a predetermined direction.

In this case, a delay time is set by estimating a representative sonic speed of a region to be diagnosed (this is referred to a set sonic speed or an assumed sonic speed). However, the sonic speed of the living body of a subject (referred to as an actual sonic speed or an optimum sonic speed) is not uniform but differs according to tissues, and there is a problem that the image quality deteriorates if the assumed sonic speed and the optimum sonic differ from each other.

Accordingly, various methods for setting the optimum sonic speed are known. For example, there is known an ultrasonic tomography apparatus capable of correcting an ultrasonic speed value set for the apparatus to improve focusing (for example, see Japanese Patent Application Laid-Open No. 8-317926).

In such an apparatus, the focus is calculated on the basis of any ultrasonic speed value inputted by an operator, and transmission and receiving of an ultrasonic wave is performed with the focus. When the operator changes the ultrasonic speed value to be inputted, the focus changes in response thereto, and the image quality of an ultrasonic image also changes. Therefore, the operator can select an ultrasonic speed value to obtain the best focus, looking at the image.

Furthermore, in such an apparatus, by determining an ultrasonic speed value which causes the amplitude of an ultrasonic received signal to be maximal, by determining an ultrasonic speed value which causes the beam width of an ultrasonic received signal to be minimal, or by determining an ultrasonic speed value which causes, for the amplitude of an ultrasonic received signal, the high-frequency component or variance of the space frequency to be maximal, the set ultrasonic speed value is corrected, and thereby focusing is improved.

However, in the prior-art technique described above, there is a problem that it is necessary to create images of multiple kinds of assumed sonic speeds (set sonic speeds), and memory, circuits, and processing time for those are required.

The present invention has been made in view of such a situation, and its object is to provide an ultrasonic diagnosis method and apparatus capable of determining the optimum sonic speed without requiring memory, circuits and processing time therefor.

SUMMARY OF THE INVENTION

In order to achieve the above object, a first aspect of the invention provides an ultrasonic diagnosis apparatus comprising: an ultrasonic probe in which a plurality of elements for transmitting an ultrasonic wave to a subject and, by receiving an ultrasonic signal reflected from the subject, outputting the received signal are arrayed; a device which changes an assumed sonic speed set in advance relative to the actual sonic speed of the ultrasonic wave to be transmitted to the subject; and an optimum sonic speed judgment device which judges a micro-structure by an RF signal obtained from the received signal by changing the assumed sonic speed and performing focusing with a delay based on the assumed sonic speed, and judges an optimum sonic speed, which is the ultrasonic speed of the subject, from phase information about the RF signal judged to be the micro-structure.

Thereby, it is possible to obtain the optimum sonic speed by using the phase change characteristic of a micro-structure signal which depends on an assumed sonic speed.

Furthermore, as shown in a second aspect, it is preferable that the optimum sonic speed judgment device judges the optimum sonic speed from amplitude information about the RF signal judged to be the micro-structure.

Thereby, it is possible to obtain the optimum sonic speed by using an amplitude change characteristic of a micro-structure signal which depends on an assumed sonic speed.

Furthermore, as shown in a third aspect, it is preferable that the optimum sonic speed judgment device judges the optimum sonic speed from phase change in the direction of the array of the elements of the ultrasonic probe, in the RF signal judged to be the micro-structure.

Furthermore, as shown in a fourth aspect, it is preferable that the optimum sonic speed judgment device judges the optimum sonic speed from phase change which depends on the assumed sonic speed, in the RF signal judged to be the micro-structure.

Furthermore, as shown in a fifth aspect, it is preferable that the optimum sonic speed judgment device judges the optimum sonic speed from amplitude change which depends on the assumed sonic speed, in the RF signal judged to be the micro-structure.

As described above, it is possible to judge the optimum sonic speed in various methods.

Furthermore, as shown in a sixth aspect, it is preferable that the optimum sonic speed judgment device uses a signal generated by changing a plurality of the assumed sonic speed from one transmission.

Furthermore, as shown in a seventh aspect, it is preferable that the optimum sonic speed judgment device uses a plurality of frames.

Thereby, it is possible to prevent displacement between frames and perform processing at a high frame rate.

Furthermore, as shown in an eighth aspect, it is preferable that the plurality of frames are obtained by a device which can generate RF data for two or more sound rays in scanning direction.

Furthermore, as shown in a ninth aspect, it is preferable that the optimum sonic speed judgment device uses such data that the resolution of the phase information is, in the direction of the array of the elements of the ultrasonic probe, equal to or more than the interval between the elements.

Thereby, it is possible to perform judgment under a high S/N ratio.

Furthermore, as shown in a tenth aspect, it is preferable that the optimum sonic speed judgment device obtains the optimum sonic speed for each of a plurality of the RF signal judged to be the micro-structures.

Furthermore, as shown in an eleventh aspect, it is preferable that the ultrasonic diagnosis apparatus according to any of the first to tenth aspects further comprises a display device which displays one or more of images which have been generated under a plurality of optimum sonic speeds obtained for a plurality of the RF signal judged to be the micro-structures, respectively.

Furthermore, as shown in a twelfth aspect, it is preferable that the display device displays an image obtained by synthesizing the images generated under the plurality of optimum sonic speeds.

Furthermore, as shown in a thirteenth aspect, it is preferable that the ultrasonic diagnosis apparatus according to the eleventh and twelfth aspect further comprises a mode switching device which switches the display mode of the display device between a normal display mode and a display mode for displaying a plurality of images being overlapped or arranged or displaying one or more images.

Similarly, in order to achieve the above object, a fourteenth aspect of the invention provides an ultrasonic diagnosis apparatus comprising: an ultrasonic probe in which a plurality of elements for transmitting an ultrasonic wave to a subject and, by receiving an ultrasonic signal reflected from the subject, outputting the received signal are arrayed; a device which changes an assumed sonic speed set in advance relative to the actual sonic speed of the ultrasonic wave to be transmitted to the subject; and an optimum sonic speed judgment device which judges an optimum sonic speed, which is the ultrasonic speed of the subject, from phase change in the direction of the array of the elements, in an RF signal obtained from the received signal by, when the assumed sonic speed is changed, performing focusing with a delay based on the assumed sonic speed, in a predetermined target area.

As described above, by setting a predetermined target area, without limiting the target area to a micro-structure, it is possible to obtain the optimum sonic speed on the basis of phase change in the target area.

Furthermore, as shown in a fifteenth aspect, it is preferable that the optimum sonic speed judgment device judges the optimum sonic speed from concave/convex change in the phase at a micro-structure, which depends on the assumed sonic speed.

As described above, it is possible to obtain the optimum sonic speed on the basis of concave/convex change in the phase, especially at a micro-structure as a predetermined target area.

Furthermore, as shown in a sixteenth aspect, it is preferable that the optimum sonic speed judgment device uses a signal generated by changing a plurality of the assumed sonic speed from one transmission.

Furthermore, as shown in a seventeenth aspect, it is preferable that the optimum sonic speed judgment device uses such data the resolution of the phase information is, in the direction of the array of the elements of the ultrasonic probe, equal to or more than the interval between the elements.

Thereby, it is possible to perform judgment under a high S/N.

Furthermore, as shown in an eighteenth aspect, it is preferable that the optimum sonic speed judgment device obtains the optimum sonic speed for each of a plurality of target areas.

Furthermore, as shown in a nineteenth aspect, it is preferable that the ultrasonic diagnosis apparatus according to any of the fourteenth to eighteenth aspects further comprises a display device which displays one or more of images which have been generated under a plurality of optimum sonic speeds obtained for a plurality of the target area, respectively.

Furthermore, as shown in a twentieth aspect, it is preferable that the display device displays an image obtained by synthesizing the images generated under the plurality of optimum sonic speeds.

Furthermore, as shown in a twenty-first aspect, it is preferable that the ultrasonic diagnosis apparatus according to any of the nineteenth or twentieth aspect further comprises a mode switching device which switches the display mode of the display device between a normal display mode and a display mode for displaying a plurality of images being overlapped or arranged or displaying one or more images.

Similarly, in order to achieve the above object, a twenty-second aspect of the invention provides an ultrasonic diagnosis method comprising: transmitting an ultrasonic wave to a subject from an ultrasonic probe in which a plurality of elements are arrayed and receiving an ultrasonic signal reflected from the subject; and judging a micro-structure by an RF signal obtained from the received signal by, when an assumed sonic speed set in advance relative to the actual sonic speed of the ultrasonic wave to be transmitted to the subject is changed, performing focusing with a delay based on the assumed sonic speed, and judging an optimum sonic speed, which is the ultrasonic speed of the subject, from phase information about the RF signal judged to be the micro-structure.

Thereby, it is possible to obtain the optimum sonic speed by using a phase change characteristic a micro-structure signal which depends on an assumed sonic speed.

Furthermore, as shown in a twenty-third aspect, it is preferable that the judgment of the optimum sonic speed is performed by judging the optimum sonic speed from amplitude information about the RF signal judged to be the micro-structure.

Thereby, it is possible to obtain the optimum sonic speed by using an amplitude change characteristic of a micro-structure signal which depends on an assumed sonic speed.

Furthermore, as shown in a twenty-fourth aspect, it is preferable that the judgment of the optimum sonic speed is performed by judging the optimum sonic speed from phase change in the direction of the array of the elements of the ultrasonic probe, in the RF signal judged to be the micro-structure.

Furthermore, as shown in a twenty-fifth aspect, it is preferable that the judgment of the optimum sonic speed is performed by judging the optimum sonic speed from phase change which depends on the assumed sonic speed, in the RF signal judged to be the micro-structure.

Furthermore, as shown in a twenty-sixth aspect, it is preferable that the judgment of the optimum sonic speed is performed by judging the optimum sonic speed from amplitude change which depends on the assumed sonic speed, in the RF signal judged to be the micro-structure.

Similarly, in order to achieve the above object, a twenty-seventh aspect of the invention provides an ultrasonic diagnosis method comprising: transmitting an ultrasonic wave to a subject from an ultrasonic probe in which a plurality of elements are arrayed and receiving an ultrasonic signal reflected from the subject; and judging an optimum sonic speed, which is the ultrasonic speed of the subject, from phase change in the direction of the array of the elements, in an RF signal obtained from a signal received from a predetermined area by, when an assumed sonic speed set in advance relative to the actual sonic speed of the ultrasonic wave to be transmitted to the subject is changed, performing focusing with a delay based on the assumed sonic speed.

Furthermore, as shown in a twenty-eighth aspect, it is preferable that the optimum sonic speed is judged from concave/convex change in the phase especially at a micro-structure as the predetermined target area, which depends on the assumed sonic speed.

As described above, according to the present invention, it is possible to obtain the optimum sonic speed by using the phase change characteristic of a micro-structure signal which depends on an assumed sonic speed.

BRIEF DESCRIPTION OF THE DRAWINGS

Fig. 1 is a system configuration diagram showing a schematic configuration of an embodiment of an ultrasonic diagnosis apparatus according to the present invention;

Fig. 2 is a graph showing the phase change characteristic of a micro-structure signal which depends on an assumed sonic speed, under the assumed sonic speed from 1400 [m/s] to 1500 [m/s];

Fig. 3 is a graph showing the phase change characteristic of a micro-structure signal which depends on the assumed sonic speed, under the assumed sonic speed from 1500 [m/s] to 1620 [m/s];

Fig. 4 is a graph showing the phase change characteristic of a plane signal which depends on the assumed sonic speed, under the assumed sonic speed from 1400 [m/s] to 1480 [m/s];

Fig. 5 is a graph showing the phase change characteristic of a plane signal which depends on the assumed sonic speed, under the assumed sonic speed from 1540 [m/s] to 1620 [m/s];

Fig. 6 is a graph showing the phase change characteristic of a speckle which depends on the assumed sonic speed, under the assumed sonic speed from 1400 [m/s] to 1480 [m/s];

Fig. 7 is a graph showing the phase change characteristic of a speckle which depends on the assumed sonic speed, under the assumed sonic speed from 1540 [m/s] to 1620 [m/s];

Fig. 8 is a graph showing the amplitude change characteristic of a micro-structure which depends on the assumed sonic speed, under the assumed sonic speed from 1400 [m/s] to 1500 [m/s];

Fig. 9 is a graph showing the amplitude change characteristic of a micro-structure signal which depends on the assumed sonic speed, under the assumed sonic speed from 1500 [m/s] to 1620 [m/s];

Fig. 10 is a diagram showing a state of receiving an ultrasonic signal;

Figs. 11A to 11C show that the curves given by the formulas (1) and (2) are in contact with each other on an (X, t) plane, wherein Fig. 11A shows the case of $V<V_0$; Fig. 11B shows the case of $V=V_0$; and Fig. 11C shows the case of $V>V_0$;

Fig. 12 is a flowchart showing the operation of an image generation section;

Fig. 13 is a flowchart showing the operation of an optimum sonic speed judgment section;

Fig. 14 is a diagram showing the relation between a value in a sonic speed judgment image and the assumed sonic speed;

Fig. 15 is a diagram showing the relation between a sonic speed judgment image value using the amplitude and the assumed sonic speed;

Fig. 16 is a flowchart showing the contents of processing by a display image generation section;

Figs. 17A and 17B are diagrams showing change in the phase of a speckle which depends on the assumed sonic speed, wherein Fig. 17A shows the case where the assumed sonic speed is faster than the optimum sonic speed, and Fig. 17B shows the case where the assumed sonic speed is slower than the optimum sonic speed;

Fig. 18 shows graphs showing histograms of concave/convex change in the phase at a micro-structure, wherein Fig. 18A shows a case where the assumed sonic speed is slower than the optimum sonic speed, and Fig. 18B shows a case where the assumed sonic speed is faster than the optimum sonic speed;

Fig. 19 is a flowchart showing a procedure for judging the optimum sonic speed from concave/convex change in the phase of the assumed sonic speed in a certain target area;

Fig. 20 is a graph showing change in unevenness of distribution which depends on the assumed sonic speed; and

Fig. 21 is a flowchart showing the flow of the processing by the display image generation section.

<u>DETAILED DESCRIPTION OF THE PREFERRED EMBODIMENTS</u>

Now, an ultrasonic diagnosis method and apparatus according to the present invention will be described below in detail with reference to accompanying diagrams.

The present invention is intended to judge a micro-structure by using the phase change characteristic of a signal shown when an ultrasonic speed (assumed sonic speed), which is set in generating a judgment image from a received ultrasonic image, is changed, and determine an optimum sonic speed, which is the ultrasonic speed of a subject, by using the phase change characteristic of the signal judged to be the micro-structure, which depends on the assumed sonic speed.

Specifically, the micro-structure refers to, for example, a minute calcification, an air bubble, or contrast medium in the subject.

In generating a judgment image by changing the ultrasonic speed to be set to multiple speeds by steps and by a predetermined amount, the ultrasonic speed to be changed to multiple speeds and set is referred to as a set sonic speed or an assumed sonic speed, relative to an actual ultrasonic speed (an actual sonic speed or an optimum sonic speed) to be transmitted to the subject, in the embodiment below.

The present invention is intended to determine the optimum sonic speed by using the phase change characteristic of a signal obtained by variously changing the assumed sonic speed at the time of generating an image.

Though what the phase change characteristic specifically is will be described in detail later, it can be simply described as below.

That is, when the assumed sonic speed is different from the optimum sonic speed, a micro-structure signal shows characteristic concave/convex phase change in the scanning direction (the direction of the array of the elements (oscillators) of an ultrasonic probe, and the amplitude/phase change characteristics of a micro-structure signal, a continuous plane/line signal and a speckle shown when the assumed sonic speed is changed are different from one another.

First, in the case of a micro-structure, when the assumed sonic speed is smaller (slower) than the optimum sonic speed (actual sonic speed), the phase convexly changes in the scanning direction, and the slope is sharper as the assumed sonic speed is closer to the optimum sonic speed. When the assumed sonic speed is larger (faster) than the optimum sonic speed (actual sonic speed), the phase concavely changes in the scanning direction, and the slope is sharper as the assumed sonic speed is closer to the optimum sonic speed. The amplitude is larger and the shape is sharper as the assumed sonic speed is closer to the optimum sonic speed.

Next, in the case of a continuous plane/line, the phase is uniform without depending on the assumed sonic speed. The amplitude is larger as the assumed sonic speed is close to the optimum sonic speed.

In the case of a speckle, both of the amplitude and the phase randomly change, depending on the assumed sonic speed.

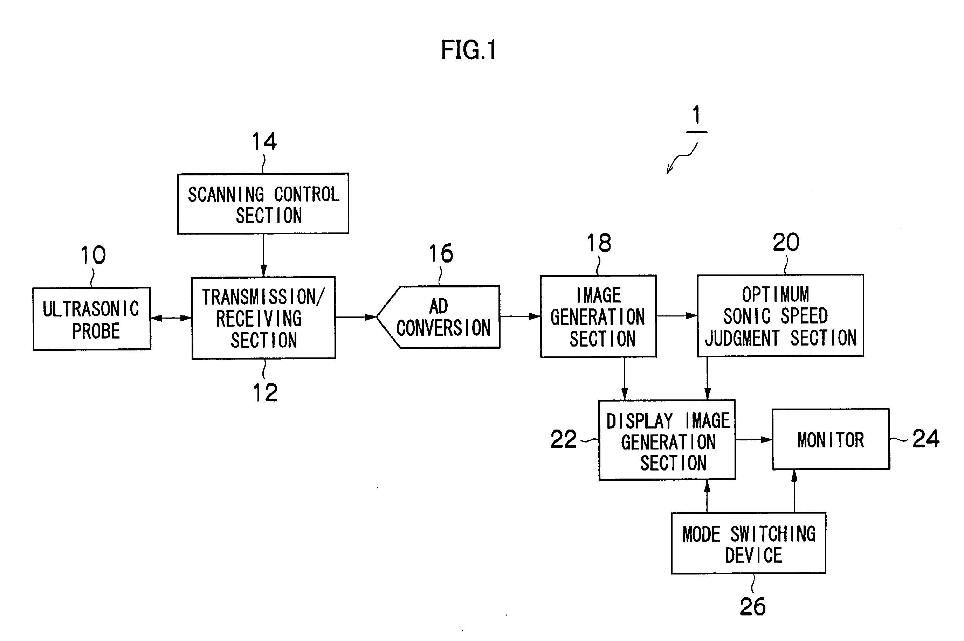

Fig. 1 is a system configuration diagram showing a schematic configuration of an embodiment of an ultrasonic diagnosis apparatus according to the present invention.

As shown in Fig. 1, an ultrasonic diagnosis apparatus 1 takes an ultrasonic image of a region of a subject to be diagnosed using an ultrasonic wave and displays it. It is configured to have an ultrasonic probe 10, a transmission/receiving section 12, a scanning control section 14, an AD conversion section 16, an image generation section 18, an optimum sonic speed judgment section 20, a display image generation section 22, a monitor 24, and a mode switching device 26.

The ultrasonic probe 10 transmits an ultrasonic wave towards a region to be diagnosed in the body of a subject and receives the ultrasonic wave reflected in the body. The ultrasonic probe 10 of this embodiment is provided with multiple ultrasonic transducers constituting a one-dimensional ultrasonic transducer array, and each ultrasonic transducer is configured, for example, by an oscillator which is a piezoelectric element, such as a PZT, with electrodes formed at both ends. These electrodes are connected to the transmission/receiving section 12 via signal lines. When voltage is applied to each electrode, the oscillator generates an ultrasonic wave. When receiving the ultrasonic wave reflected, the oscillator generates an electrical signal, and this electrical signal is outputted as a received signal.

The transmission/receiving section 12 gives an ultrasonic transmitted signal to the ultrasonic probe 10 to cause ultrasonic waves from the oscillators, and transmits the waves on the basis of a delay given by the scanning control section 14. Then, the transmission/receiving section 12 receives the reflected ultrasonic waves, and amplifies a received signal of each element which has been outputted by the ultrasonic probe 10 as it is (without performing focusing of the received waves).

The AD conversion section 16 receives the ultrasonic received signal from the transmission/receiving section 12, performs AD-conversion of them, and hands them over to the image generation section 18. The image generation section 18 stores the received data which has been received from the AD conversion section 16. The image generation section 18 generates, from the stored received data of each element, RF data for which received-data focusing has been performed with a delay based on sonic speeds variously set (referred to as assumed sonic speeds relative to an actual sonic speed (actual sonic speed) for transmitting a wave to a subject) and which is based on each assumed sonic speed. This will be described in detail later.

The optimum sonic speed judgment section 20 judges the optimum sonic speed from the RF data generated by the image generation section 18, observing phase change in the direction of scanning of a micro-structure.

The display image generation section 22 generates a display image to be displayed on the monitor 24 from the images generated by the image generation section 18 and a result of judgment of a judgment image generated by the optimum sonic speed judgment section 20. The mode switching device 26 switches modes for displaying an image on the monitor 24.

Next, before describing the operation of the above ultrasonic diagnosis apparatus 1, a phase change characteristic shown when the assumed sonic speed is changed will be described.

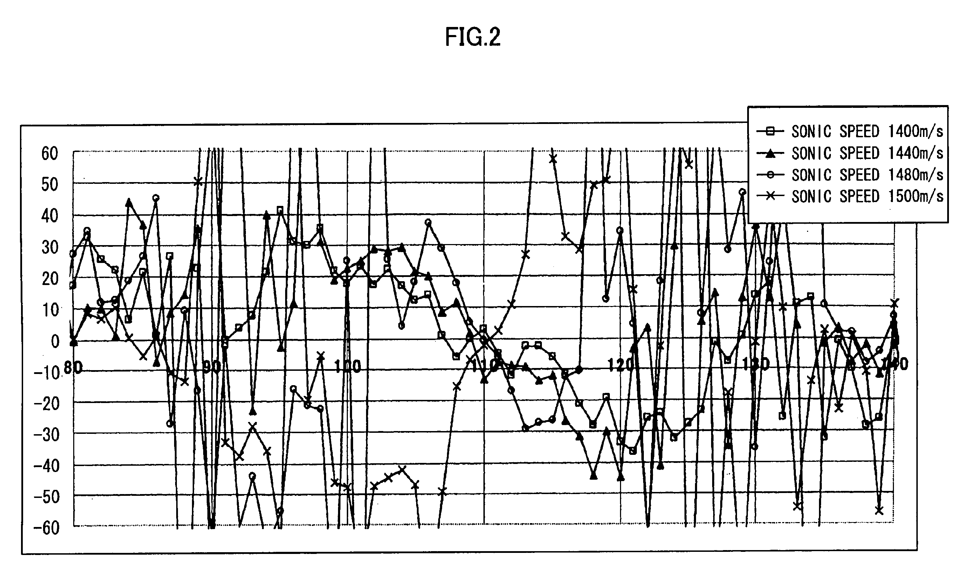

Figs. 2 to 9 show graphs indicating the phase change characteristic shown when the assumed sonic speed is changed.

Each graph shows the phase change characteristic shown when the assumed sonic speed is changed by increment of 40 [m/s] or 20 [m/s] from about 1400 [m/s] up to 1620 [m/s], with the axis of abscissas as the scanning direction (X position) and the axis of ordinates as the phase.

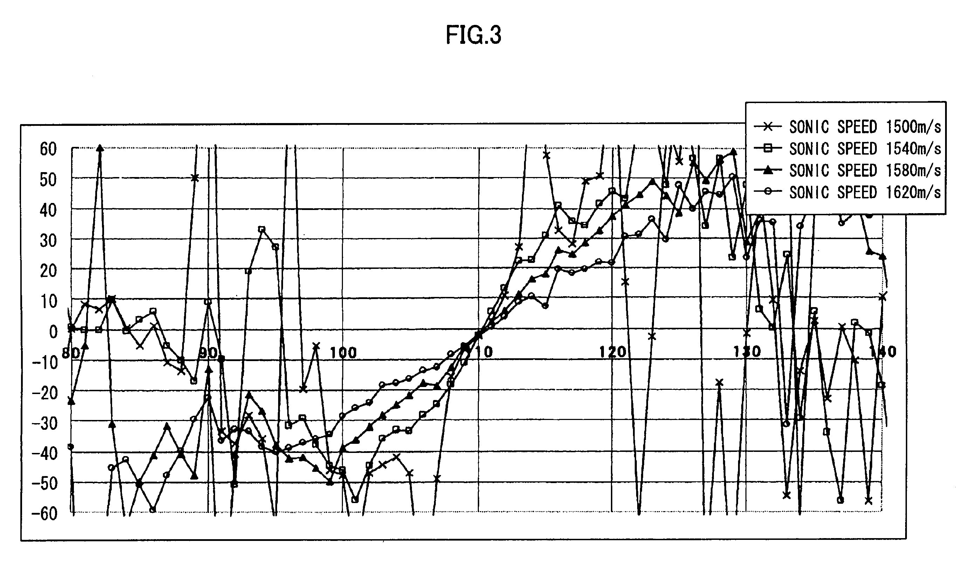

Fig. 2 is a graph showing the phase change characteristic of a micro-structure which depends on the assumed sonic speed, under the assumed sonic speed from 1400 [m/s] to 1500 [m/s]. Fig. 3 is a graph showing the phase change characteristic of a micro-structure signal which depends on the assumed sonic speed, under the assumed sonic speed from 1500 [m/s] to 1620 [m/s].

In the case of Fig. 2, in which the assumed sonic speed is from 1400 [m/s] to 1500 [m/s], the graph of the assumed sonic speed of 1500 [m/s] shows a positive slope in the vicinity of the X position of 100 to 120. In all the other graphs, in which the assumed sonic speed is smaller than 1500 [m/s] (that is, the assumed sonic speed is slower), a downward slope is shown. The slope is sharper as the assumed sonic speed is closer to 1500 [m/s], and the slope is gentler as the assumed sonic speed is slower than 1500 [m/s].

In the case of Fig. 3, in which the assumed sonic speed is from 1500 [m/s] to 1620 [m/s], all the graphs show an upward slope in the vicinity of the X position of 100 to 120. When the assumed sonic speed is 1500 [m/s], the slope is the sharpest. As the assumed sonic speed is larger than 1500 [m/s], the slope is gentler.

The shapes of the graphs in Figs. 2 and 3 are thought to show that a micro-structure exists in the vicinity of the X position of 100 to 120.

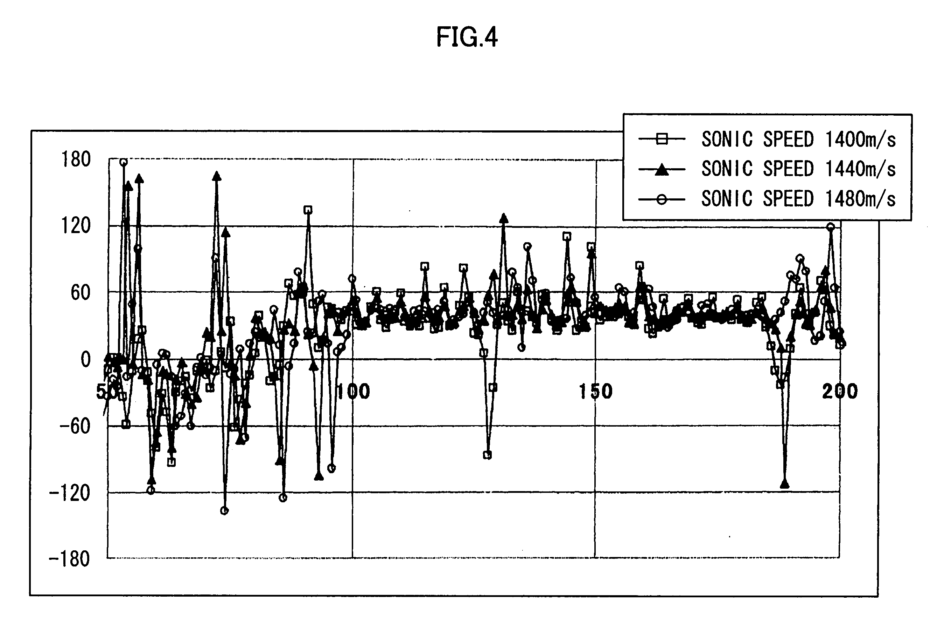

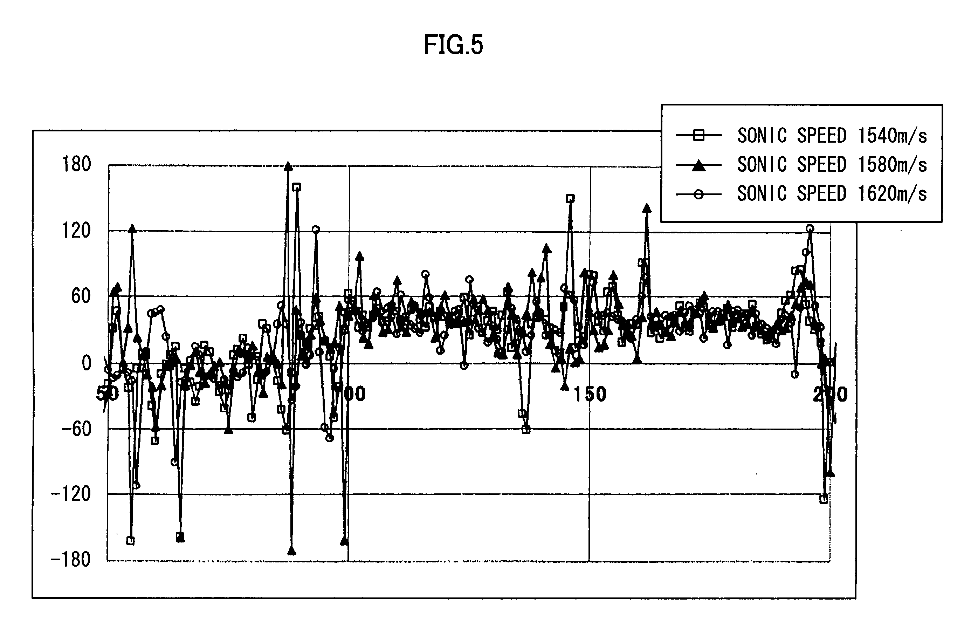

Fig. 4 is a graph showing the phase change characteristic of a plane signal which depends on the assumed sonic speed, under the assumed sonic speed from 1400 [m/s] to 1480 [m/s]. Fig. 5 is a graph showing the phase change characteristic of a plane signal which depends on the assumed sonic speed, under the assumed sonic speed from 1540 [m/s] to 1620 [m/s].

As seen from Figs. 4 and 5, the phase does not change much even when the assumed sonic speed is changed, in the vicinity of the X position of 100 to 130 and 150 to 180 in all the graphs. It can be thought that this is because a plane (a continuous plane) exists in that part.

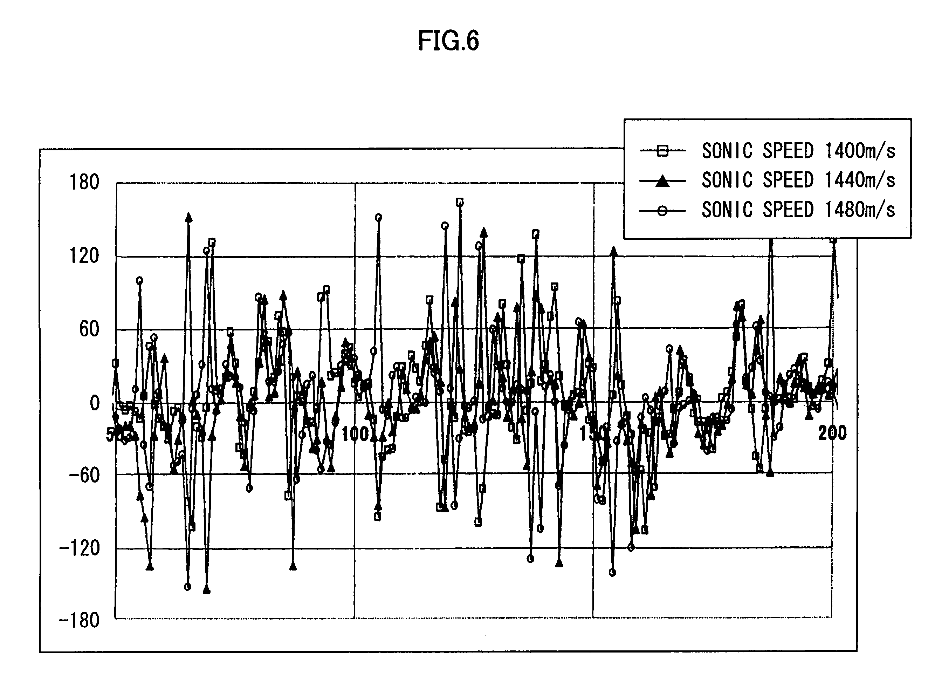

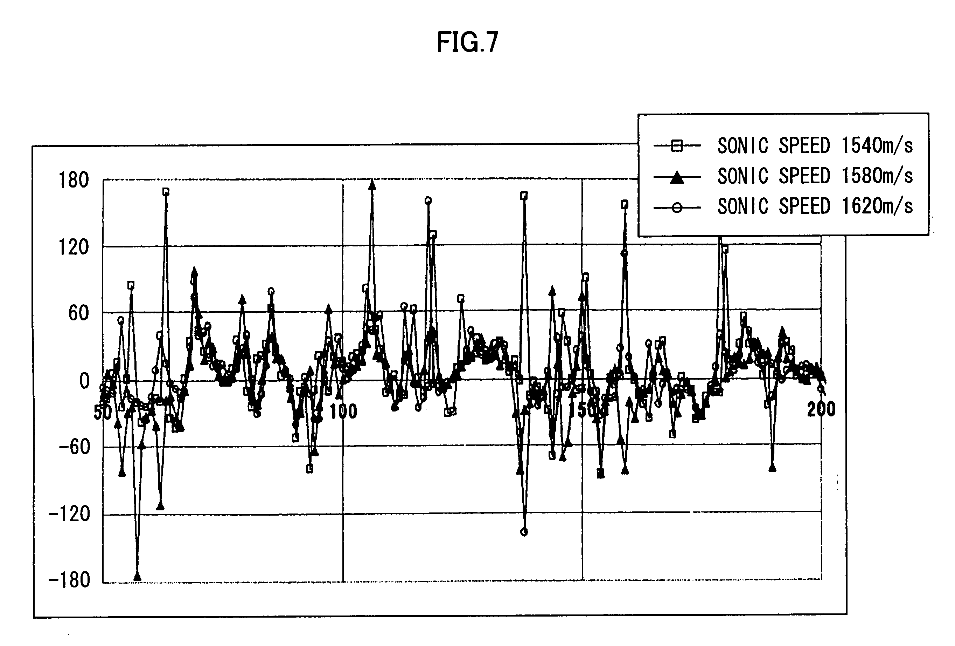

Fig. 6 is a graph showing the phase change characteristic of a speckle which depends on the assumed sonic speed, under the assumed sonic speed from 1400 [m/s] to 1480 [m/s], and Fig. 7 is a graph showing the phase change characteristic of a speckle which depends on the assumed sonic speed, under the assumed sonic speed from 1540 [m/s] to 1620 [m/s]. In the case of a speckle, the phase randomly changes when the assumed sonic speed is changed, as shown in Figs. 6 and 7.

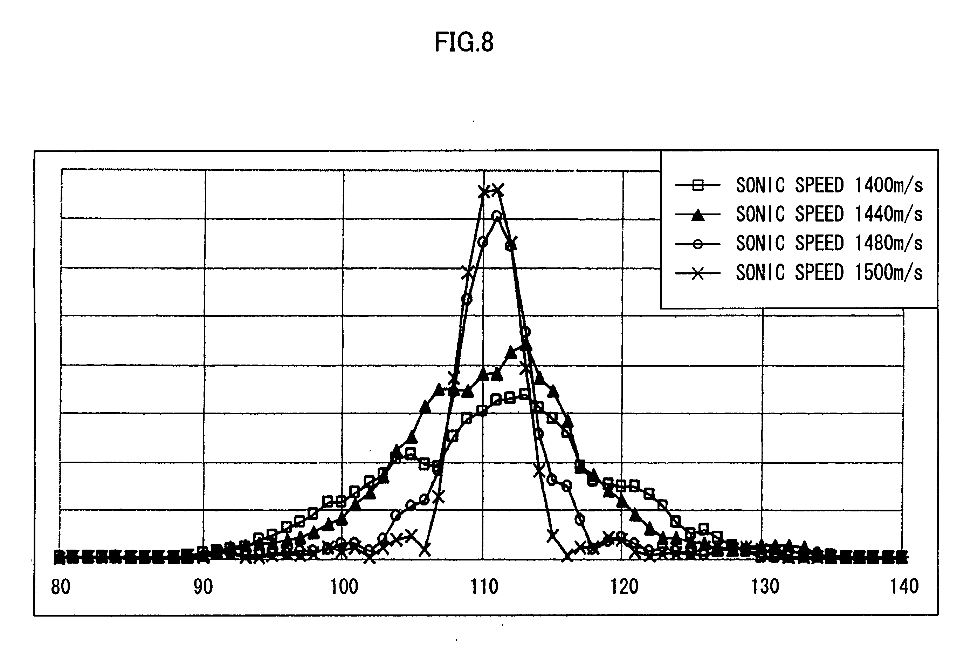

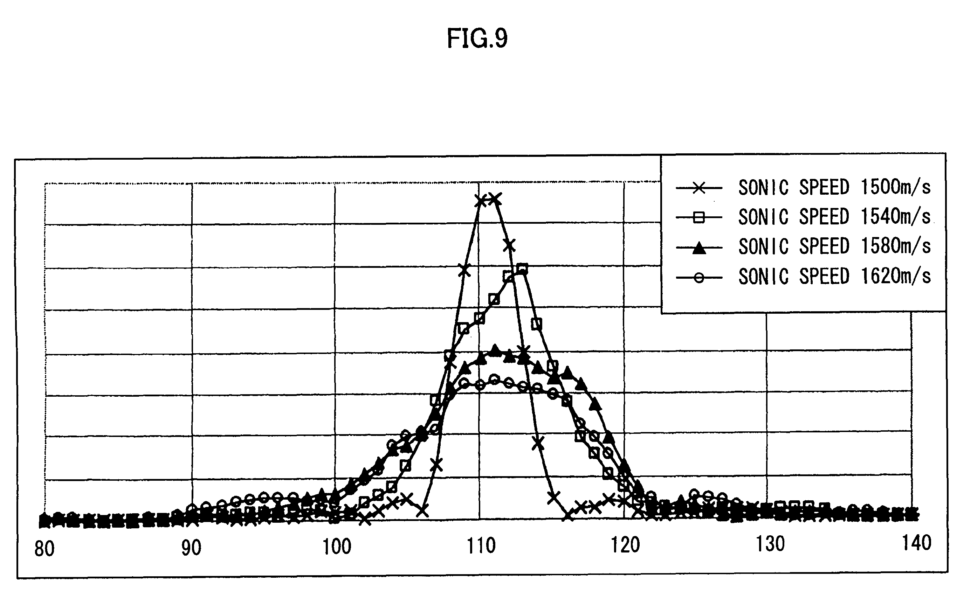

Fig. 8 is a graph showing the amplitude change characteristic of a micro-structure which depends on the assumed sonic speed, under the assumed sonic speed from 1400 [m/s] to 1500 [m/s]. Fig. 9 is a graph showing the amplitude change characteristic of a micro-structure which depends on the assumed sonic speed, under the assumed sonic speed from 1500 [m/s] to 1620 [m/s].

As seen from Figs. 8 and 9, all of the graphs showing change in the amplitude of a micro-structure are mountain-shaped (upwardly convex) graphs having a top (the maximum value) in the vicinity of the X position of 110. The amplitude value is maximal when the assumed sonic speed is 1500 [m/s], and the maximum amplitude value is larger and the shape is sharper as the assumed sonic speed is closer to 1500 [m/s].

Next, description will be made on the reason why the phase change of a micro-structure shown when the assumed sonic speed is changed has the characteristic as shown in Figs. 2 and 3 as graphs.

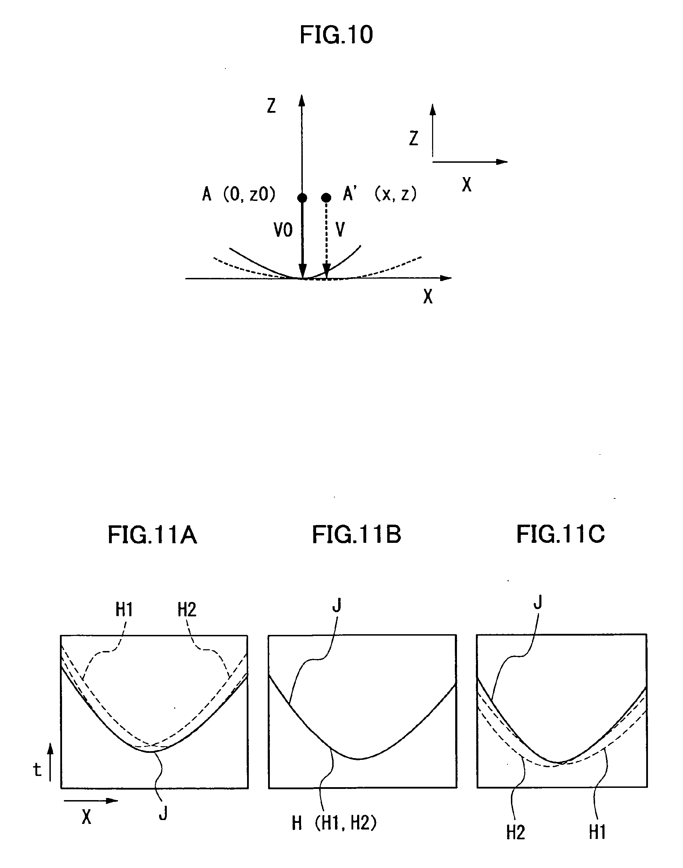

It is assumed that, in Fig. 10, an ultrasonic wave reflected from a point A $(0, z_0)$ under a sonic speed $V_0$ is observed at time t after the reflection, at an element (oscillator) at a position X in the ultrasonic probe 10. Then, the time t is calculated as in the following formula (1):

$$t = \mathrm{sqrt}(z_0{}^2 + X^2)/V_0 \; ...(1)$$

In the formula (1), sqrt() means that the square root of the value in the parentheses is taken.

It is also assumed that, in Fig. 10, an ultrasonic wave reflected from a point A'(x, z) under a sonic speed V is similarly observed at the time t after the reflection, at the element (oscillator) at the position X in the ultrasonic probe 10. Similarly to the above, the time t is indicated by the following formula (2):

$$t = \mathrm{sqrt}\{z^2 + (X-x)^2\}/V \; ...(2)$$

The locus of the point A' in the case where curves given by the formulas (1) and (2) are in contact with each other on a plane (X, t) is given by the formula (3):

$$z^2 = x^2 \times \{V^2/(V_0{}^2 - V^2)\} + z_0{}^2 V^2/V_0{}^2 \; ...(3)$$

The position A' indicates a position where the signal strengthens when the phase is adjusted as an assumed sonic speed V, relative to the optimum sonic speed (actual sonic speed), and addition is performed.

From the formula (3), the locus of the position A' (x, z) forms an ellipse with the origin as the center in the case of $V>V_0$, and forms a hyperbola with the origin as the center in the case of $V<V_0$. In the case of a B-mode image, the downward direction of the z-axis indicates upward, the locus of the ellipse with the origin as the center in the case of $V>V_0$ is assumed to be a concave shape, and the locus of the hyperbola with the origin as the center in the case of $V<V_0$ is assumed to be a convex shape.

Figs. 11A to 11C show that the curves given by the formulas (1) and (2) are in contact with each other on the (X, t) plane. Fig. 11A shows the case of $V<V_0$. An solid line J indicates a wave reflected from the point A, and a broken line H1 indicates a wave reflected from the position A' in the case where the position A' is on the right side of the z-axis as in Fig. 10. A broken line H2 indicates a light reflected from a point on the left side of the z-axis in Fig. 10 though the point is not shown. Because $V<V_0$ is assumed now, the time t of the wave reflected from the position A' is larger as for the same X position. Therefore, the broken line H1 (H2) is shown on the upper side of the solid line J.

Fig. 11B shows the case of $V=V_0$, and Fig. 11C shows the case of $V>V_0$. When V comes closer to $V_0$, the

broken line H comes closer to the solid line J. In the case of $V=V_0$, the broken line H corresponds to the solid line J. In the case of Fig. 11C, a broken line appears on the lower side of a solid line, contrary to the case of Fig. 11A.

From these figures, it can be intuitively understood that the lotus of the point A' (x, z) where the above-described broken line is in contact with the above-described solid line has the inclination as described above.

Though an observed reflected wave is simply assumed to be a line in the model described here, it is actually necessary to take it into consideration that the waveform has a width toward the t direction and that there is strength difference in the X direction. Furthermore, though it is assumed in this model that reflections from the point A and the point A' occur at the same time for simplification, it is actually necessary to take into consideration the time required until reflection occurs after an ultrasonic wave is transmitted to each of the points.

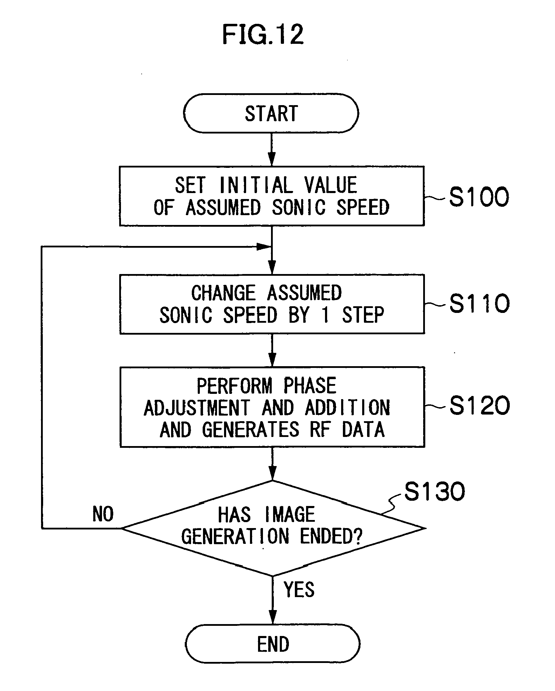

Next, the operation of the image generation section 18 in the apparatus configuration in Fig. 1 will be described along the flowchart of Fig. 12.

The image generation section 18 generates an image from data obtained under various sonic speeds set by changing the assumed sonic speed.

First, at step S 100 in Fig. 12, an initial value of the assumed sonic speed to be variously changed is set. This value is not especially limited, and any value can be set. For example, 1400 [m/s] can be set as in the example in Fig. 2 described above.

Then, a signal is sent to the ultrasonic probe 10 from the transmission/receiving section 12 controlled by the scanning control section 14 under the set initial value, and data under the initial value of the assumed sonic speed is acquired and sent to the image generation section 18.

Next, at step S 110, the assumed sonic speed is changed by one step of a predetermined amount, and ultrasonic data is acquired under the changed assumed sonic speed. This one step of a predetermined amount is not especially limited. For example, 40 [m/s] is possible as in the example in Fig. 2, and 10 [m/s] or 20 [m/s] may be also possible. The assumed sonic speed is changed by the predetermined amount.

Next, at step S120, by performing addition of the data obtained under the assumed sonic speeds after adjusting the phases, RF (radio frequency) data is generated. This RF data includes both of amplitude information and phase information. In this way, the RF data is created with images under all the assumed sonic speeds.

Then, at step S130, it is judged whether image generation has ended. If it has not ended yet, then the flow returns to step S 110, where the assumed sonic speed is changed by one step again. Then, image generation is continued. Whether image generation has ended is judged on the basis of whether the processing for all the assumed sonic speeds has ended or not. The judgment can be performed, for example, by determining how many steps the assumed sonic speed can be changed before the processing ends, in advance, and counting the number of times of the assumed sonic speed being changed.

Next, the operation of the optimum sonic speed judgment section 20 will be described.

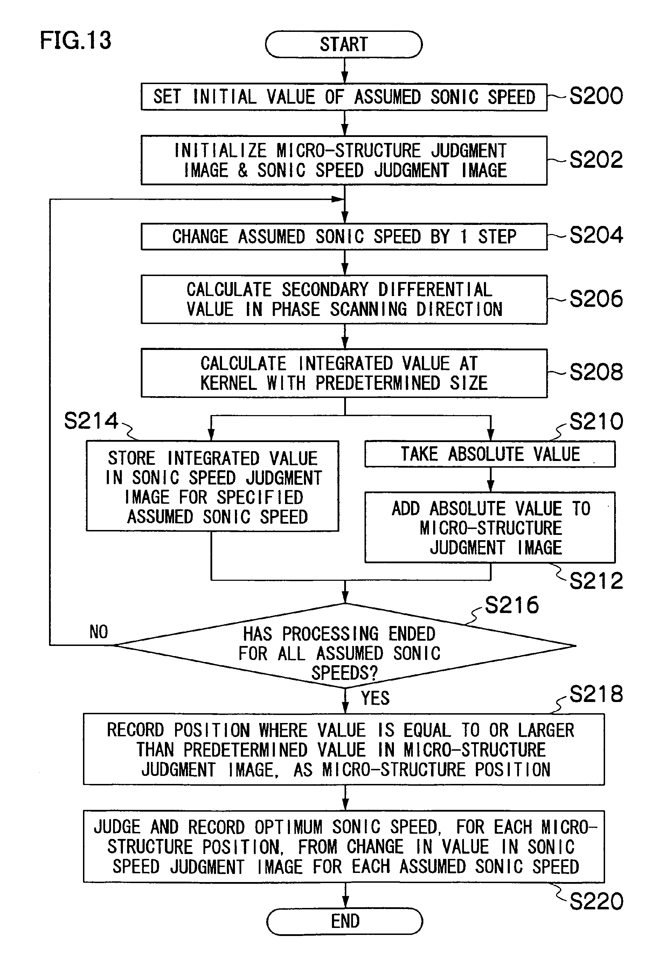

Fig. 13 is a flowchart showing the flow of optimum sonic speed judgment processing by the optimum sonic speed judgment section 20.

First, at step S200 in Fig. 13, an initial value of the assumed sonic speed is set. Data which has been already obtained in the processing by the image generation section 18 can be used as the value. Next, at step S202, the value of each pixel of a micro-structure judgment image and a sonic speed judgment image is initialized as 0.

Next, at step S204, the assumed sonic speed is changed by one step and data under that sonic speed is acquired. As for this data also, data which has been already obtained in the processing by the image generation section 18 can be used.

Next, at step S206, a secondary differential value in the phase scanning direction is calculated from the data under the assumed sonic speed. The scanning direction corresponds to the direction of the array of the oscillators (elements) of the ultrasonic probe 10.

Next, at step S208, the secondary differential value is integrated at a kernel with a predetermined size to calculate an integrated value. The size of the kernel is not especially limited. The size of 9x4, 16x8 or the like is used according to the resolution.

Next, generation of a micro-structure judgment image and generation of a sonic speed judgment image are performed at the same time in parallel.

At step S210, the absolute value of the integrated value which has been calculated is taken, and, at the next step S212, the absolute value of the integrated value is added to the micro-structure judgment image.

In this way, by adding the values obtained by integrating the secondary differential values under all the assumed sonic speeds to the micro-structure judgment image set to the default of 0 at the beginning, a micro-structure judgment image is created. In the case of a micro-structure, the secondary differential value is a positive value when the assumed sonic speed is faster than the optimum sonic speed, and it is a negative value when the assumed sonic speed is slower than the optimum sonic speed. Therefore, when the secondary differential values under the assumed sonic speeds are integrated at a predetermined kernel, the signal is strong only at the micro-structure.

Therefore, the micro-structure judgment image generated by adding the values is an image in which the signal is strong only at the micro-structure, and thereby, it is possible to judge the micro-structure.

Meanwhile, at step S214, the value integrated at the kernel with a predetermined size is stored in a sonic speed judgment image for a specified assumed sonic speed.

At the next step S216, it is judged whether the above processing has ended for all the assumed sonic speeds. If it has not ended yet, then the flow returns to step S204, changes the assumed sonic speed by one step, and repeats the processing for the next assumed sonic speed. When the processing has ended for all the assumed sonic speeds, a position where the value is equal to or larger than a predetermined value in the micro-structure judgment image is recorded as a micro-structure position at the next step S218.

Lastly, at step S220, the optimum sonic speed is judged for each micro-structure position, from the change in the value in the sonic speed judgment image for each assumed sonic speed, and it is recorded.

Net, a method for judging the optimum sonic speed will be described in detail.

One or more kinds of assumed sonic speeds are used for judgment of the optimum sonic speed. In the flowchart of Fig. 13, it is shown that the micro-structure judgment image processing and the sonic speed judgment image processing are performed under the same assumed sonic speed through steps S204 to S214. However, the kinds and the number of the assumed sonic speeds used for the micro-structure judgment and the kinds and the number of the assumed sonic speeds used for the optimum sonic speed judgment are not necessary required to be the same.

Furthermore, the micro-structure judgment method is not limited to that indicated by the flowchart in Fig. 13, and other methods are possible. For example, a method is also possible in which the sign is reversed when the assumed sonic speed is slower than the optimum sonic speed, without the absolute value of an integrated value being taken. The present invention is originally intended to determine the optimum sonic speed. Though the optimum sonic speed is not known yet, it is possible to use this method by using an assumed sonic speed equal to or slower than a predetermined speed and an assumed sonic equal to or faster than the predetermined speed. Thus, it is possible to generate a judgment image even if the optimum sonic speed is not known.

In addition, the following methods can be also used: a method in which only signs are integrated, a method in which uniformity is quantified to be an indicator, such as the variance of secondary differential values and inclination, a method in which a concave/convex shaped filter corresponding to a phase shift is applied, and cross-correlation with a phase or a waveform image is taken and extracted, and the like.

The micro-structure judgment may be specified by a user.

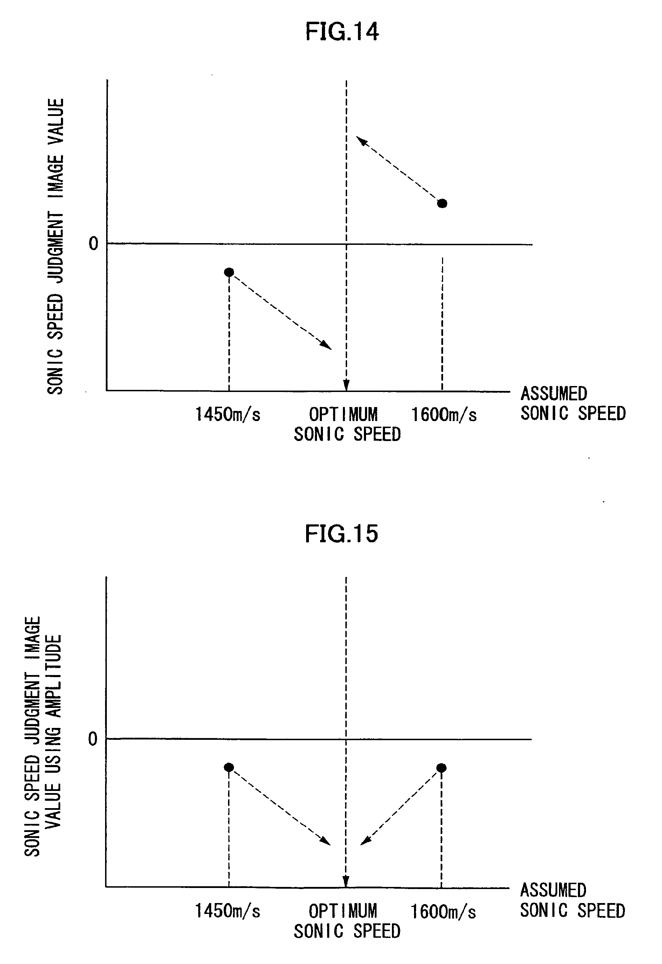

Fig. 14 shows the relation between the assumed sonic speed and the value at a micro-structure position in a sonic speed judgment image, in the optimum sonic speed judgment.

As shown in Fig. 14, if the assumed sonic speed is slower than the optimum sonic speed, the value in the sonic speed judgment image is a negative value, and, if the assumed sonic speed is faster than the optimum sonic speed, the value in the sonic speed judgment image is a positive value. Furthermore, in both of the cases, as the assumed sonic speed is closer to the optimum sonic speed, the inclination is sharper. Therefore, the value in the sonic speed judgment image, which is the result of integration of the assumed sonic speed, is a smaller value when it is a negative value, and a larger value when it is a positive value. In this way, the value in the sonic speed judgment image is generally uniquely determined depending on whether the assumed sonic speed is slower or faster than the optimum sonic speed or depending on the amount of deviation.

Therefore, the optimum sonic speed can be judged from the value of at least one kind of assumed sonic speed. The relation between the amount of deviation from the optimum sonic speed and the value in the sonic speed judgment image may be experimentally determined in advance and set in a table or may be given by a formula.

In the case of using two or more kinds of assumed sonic speeds, a method of taking a weighted average of optimum sonic speed values determined from values under the assumed sonic speeds is possible, or a method is also possible in which optimum sonic speed values are assumed, and such an optimum sonic speed value that the weighted square error between the value determined from a table or a formula for each assumed sonic speed, and the actual value is minimal.

It is possible to exclude a position where the relation between the assumed sonic speed and the value in the judgment image is deviated from a value determined from a table or a formula by an amount larger than a predetermined amount, without regarding the position as a micro-structure.

As other methods for judging the optimum sonic speed which can be used, there are a method using the maximum or minimum value, instead of the integrated value of a secondary differential value at a kernel, or a method in which correlation between the phase or the waveform image under each assumed sonic speed and a concave/convex shaped filter prepared in advance is taken to perform optimum concave/convex judgment.

Furthermore, by using the amplitude change characteristic as shown in Figs. 8 and 9 together, the accuracy of the optimum sonic speed judgment can be improved.

Fig. 15 shows the relation between the assumed sonic speed and the value in a sonic speed judgment image using the amplitude.

As shown in Fig. 15, a judgment image value using amplitude information is bilaterally symmetric with the optimum sonic speed value as the center. In this case, because of the characteristic that the convex shape is sharper as the value is closer to the optimum sonic speed, as described above, a method similar to the above-described method using a phase can be used. However, since the convex-shaped change in the amplitude does not depend on whether the sonic speed is faster or slower than the optimum sonic speed, it is not possible to judge whether fast or slow only by the change. Therefore, multiple frames may be used for the optimum sonic speed judgment.

Recent software-based ultrasonic apparatuses and the high-performance circuit configuration of analog-based ultrasonic apparatuses make it possible to generate images under various assumed sonic speeds from a received signal obtained via the same one transmission. In the configuration of the present apparatus, images under various assumed sonic speeds can be obtained without frame displacement. Consequently, it is possible to judge the optimum sonic speed with a high accuracy.

Furthermore, by using phase information which can be highly resolved in the scanning direction, it is possible to judge the optimum sonic speed with a higher accuracy. Inversion by $\pm 180°$ does not easily occur. Furthermore, by using multiple frames, it is possible to judge the optimum sonic speed with a higher accuracy. Furthermore, by using received data acquired via the same one transmission, it is possible to judge the optimum sonic speed with a higher accuracy.

The phase change characteristic of a micro-structure is expressed by scanning-direction concave/convex change here. However, it can be expressed as phase change according to assumed sonic speeds at the same position. The optimum sonic speed judgment can be used for any of the characteristics.

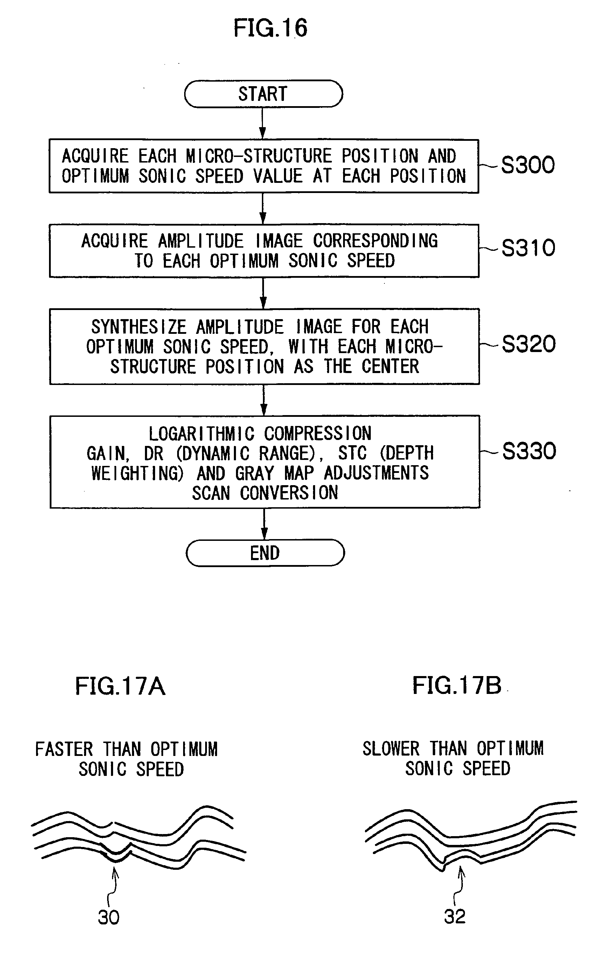

Fig. 16 is a flowchart showing the flow of processing by the display image generation section 22.

First, at step S300 in Fig. 16, each micro-structure position and the optimum sonic speed value at each position are acquired.

Next, at step S310, an amplitude image corresponding to the optimum sonic speed value at each position is acquired.

Next, at step S320, the amplitude image for each optimum sonic speed is synthesized, with each micro-structure position as the center. A method for the synthesis is as follows. A rectangular or circular area, or an area of any shape determined by specification by a user or a brightness level is set so that each image is overlapped with other images, with each micro-structure position as the center. At an overlapped area, an amplitude image is added at a rate corresponding to the distance from each micro-structure position.

Though an example of synthesizing multiple optimum sonic speed images has been described here, it is also possible to simply arrange multiple images or select an image of an average sonic speed. It is also possible to limit those to be used, among the multiple micro-structures.

Next, at step S330, the result is logarithmically compressed, and gain, DR (dynamic range), STC (depth weighting) and gray map adjustments are made therefor. Furthermore, scan conversion is performed to generate a display image.

As for the display mode of the image, images generated under multiple optimum sonic speeds may be individually displayed, or multiple of them may be displayed. It is also possible to display an image obtained by synthesizing the images generated under the multiple optimum sonic speeds.

Furthermore, it is also possible to switch the image display mode among a normal display mode and other display modes by the mode switching device 26.

As described above, according to this embodiment, the relation between the assumed sonic speed of a micro-structure signal and the phase is generally uniquely determined by whether the assumed sonic speed is faster or slower than the optimum sonic speed or by the amount of deviation. Therefore, if there is at least one kind of assumed sonic speed data, the optimum sonic speed can be determined. As a result, it is possible to obtain the optimum sonic speed with a little memory, a few circuits and a little processing time.

As described above, according to this embodiment, it is possible to obtain the optimum sonic speed by using the phase change characteristic of a micro-structure which depends on the assumed sonic speed.

In the case of a complete speckle, the phase randomly changes, depending on the assumed sonic speed. In the case of a continuous plane, the phase is constant without depending on the assumed sonic speed. On the other hand, the phase of a micro-structure shows concave/convex change in the scanning direction, and the concave/convex shape changes according to the assumed sonic speed. The inside of a living body is considered to be configured not by complete speckles or complete continuous planes with a smooth surface where the reflection from each part is the same, but by speckles which locally includes strong reflections.

Fig. 17 shows phase change of a speckle which depends on the assumed sonic speed. Fig. 17A shows the case of the assumed sonic speed faster than the optimum sonic speed, and Fig. 17B shows the case of the assumed sonic speed slower than the optimum sonic speed. In this case, a downwardly convex shape 30 (an isolated point) locally appears, as shown in Fig. 17A. In Fig. 17B, an upwardly convex shape 32 (an isolated point) locally appears.

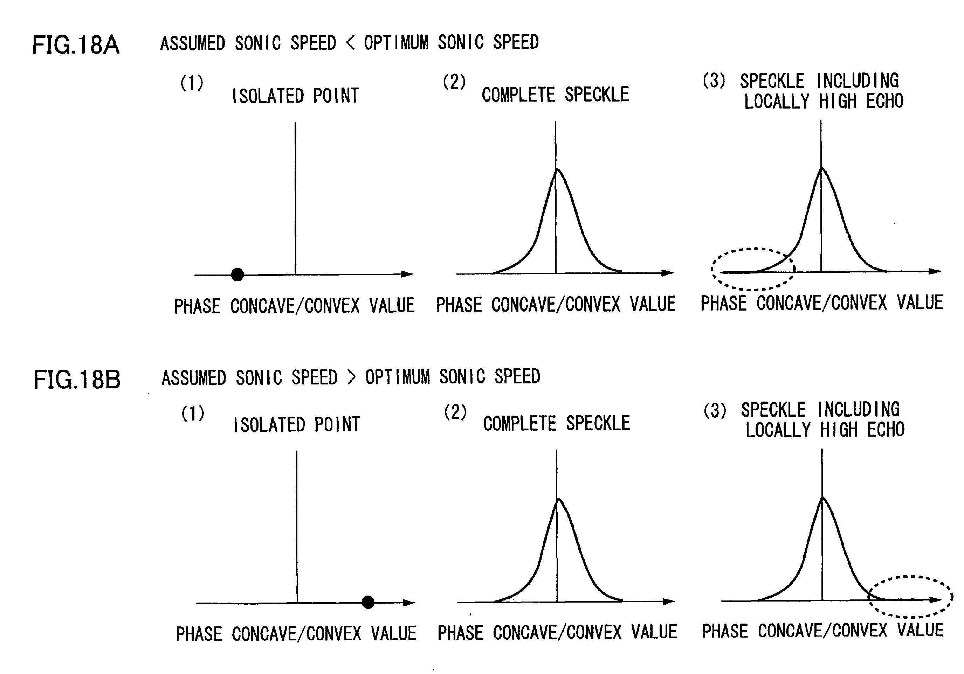

Fig. 18 shows histograms of concave/convex change in the phase at a micro-structure. Fig. 18A shows a case where the assumed sonic speed is slower than the optimum sonic speed, and Fig. 18B shows a case where the assumed sonic speed is faster than the optimum sonic speed. Figs. 18(1), 18(2) and 18(3) show the cases of an isolated point, a complete speckle and a speckle where high echoes are locally mixed, respectively.

As shown in Fig. 18A(1), when the assumed sonic speed is slower than the optimum sonic speed, the phase concave/convex value of the isolated point appears on the negative side. As shown in Fig. 18B(1), when the assumed sonic speed is faster than the optimum sonic speed, the concave/convex value of the isolated point appears on the positive side.

In the case of a complete spackle, the phase randomly changes. Therefore, when the histogram of the phase concave/convex values is taken, the histogram forms a generally normal distribution as shown in Fig. 18A(2) and Fig. 18B(2).

In the case of a speckle where high echoes are locally mixed, when the assumed sonic speed is slower than the optimum sonic speed, the histogram of the phase concave/convex values shows uneven distribution on the negative side as shown in Fig. 18A(3). On the other hand, when the assumed sonic speed is faster than the optimum sonic speed, the histogram of the phase concave/convex values shows uneven distribution on the positive side as shown in Fig. 18B(3). In this way, the optimum sonic speed can be determined from uneven distribution of the concave/convex change in the phase.

Now, description will be made on an example of judging the optimum sonic speed from concave/convex change in the phase of the assumed sonic speed in a certain target area.

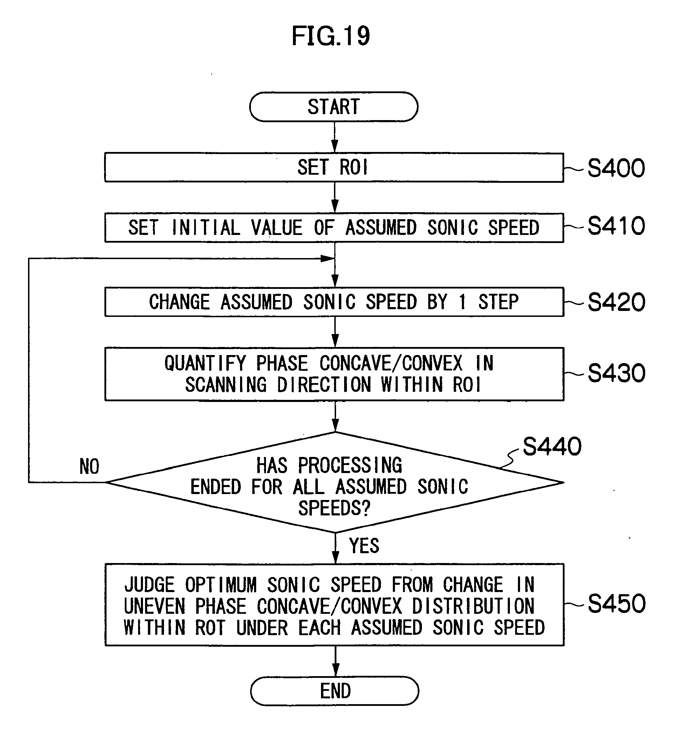

Fig. 19 shows a procedure for judging the optimum sonic speed from concave/convex change in the phase of the assumed sonic speed in a certain target area.

First, at step S400 in Fig. 19, a target area (ROI; Region Of Interest) is set. Setting of each target area may be specified by a user. It is also possible to set multiple target areas obtained by dividing the full screen into areas with a predetermined size.

Next, at step S410, an initial value of the assumed sonic speed to be variously changed is set, similarly to the above-described step S100 in Fig. 12. This value is not especially limited, and any value can be set. For example, 1400 [m/s] can be set as in the example shown in Fig. 2. Then, a signal is sent to the ultrasonic probe 10 from the transmission/receiving section 12 controlled by the scanning control section 14 under the set initial value, and data under the initial value of the assumed sonic speed is acquired and sent to the image generation section 18.

Next, at step S420, the assumed sonic speed is changed by one step of a predetermined amount, and ultrasonic data is acquired under the changed assumed sonic speed. This one step of a predetermined amount is not especially limited. For example, 20 [m/s] is possible as in the example in Fig. 2, and other values such as 10 [m/s] may be also possible. The assumed sonic speed is changed by the predetermined amount.

Next, at step S430, the phase concaves and convexes in the scanning direction in the target areas are quantified. The quantification of the phase concaves and convexes is not especially limited. For example, an integrated value of a secondary differential value or a value of correlation with a concave/convex pattern may be used. Waveform addition along the concaves and convexes is also possible.

Next, at step S440, it is judged whether the processing for all the assumed sonic speeds has ended or not. If the processing for all the assumed sonic speeds has not ended yet, the flow returns to step S420, where the assumed sonic speed is change by one step. Then, the above processing is repeated.

When the processing for all the assumed sonic speeds has ended, the optimum sonic speed is judged from change in uneven distribution of the phase concaves and convexes in the target area under each assumed sonic speed, at the next step S450.

Evaluation of unevenness of distribution can be performed with the use of an average of the maximum and minimum values of the phase concaves and convexes, an average of a predetermined number, or difference among absolute values.

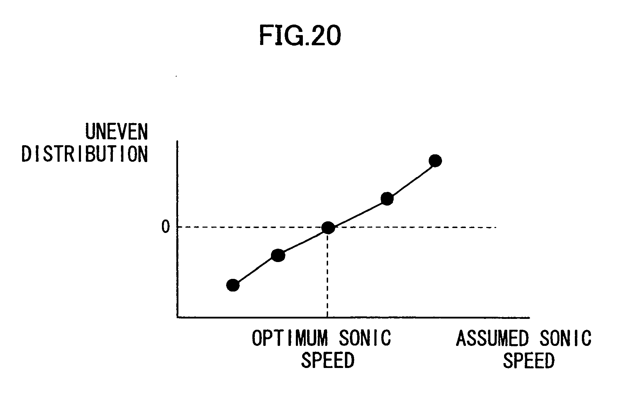

Fig. 20 shows change in unevenness of distribution which depends on the assumed sonic speed. As shown in Fig. 20, such an assumed sonic speed that the unevenness is the closest to 0 is judged to be the optimum sonic speed. Alternatively, an intermediate value of such assumed sonic speeds that unevenness on the positive and negative sides is almost the same may be judged to be the optimum sonic speed.

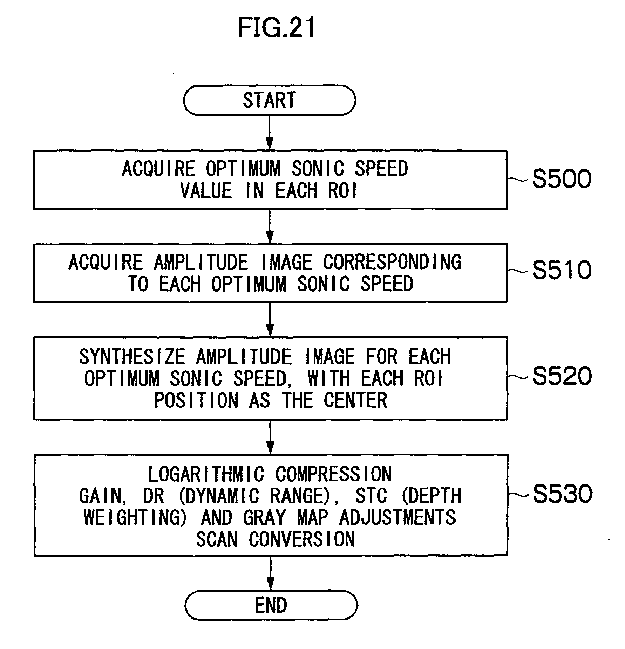

Fig. 21 is a flowchart showing the flow of processing by the display image generation section 22, similarly to Fig. 16 described above. As shown in Fig. 21, an optimum sonic speed image may be generated from the optimum sonic speed value of each target area.

First, at step S500 in Fig. 21, an optimum sonic speed value in each target area is acquired. Next, at step S510, an amplitude image corresponding to each optimum sonic speed is acquired. Next, at step S520, the amplitude image for each optimum sonic speed is synthesized, with each target area position as the center.

Next, at step S530, the result is logarithmically compressed, and gain, DR (dynamic range), STC (depth weighting) and gray map adjustments are made therefor. Furthermore, scan conversion is performed to generate a display image.

As for the display mode of the image, images generated under multiple optimum sonic speeds may be individually displayed, or multiple of them may be displayed. It is also possible to display an image obtained by synthesizing the images generated under the multiple optimum sonic speeds.

Furthermore, it is also possible to switch the image display mode among a normal display mode and other display modes by the mode switching device 26.

According to the example described above, it is possible to judge whether the assumed sonic speed is faster or slower than the optimum sonic speed or how much the assumed sonic speed is deviated, from unevenness of distribution of concave/convex change in the phase of a speckle, and therefore, it is possible to judge the optimum sonic speed from the judgment.

The ultrasonic diagnosis method and apparatus of the present invention has been described in detail. The present invention is not limited to the above example, and it goes without saying that various kinds of improvements and variations can be made within the range not deviating from the spirit of the present invention.

**Claims**

1. An ultrasonic diagnosis apparatus (1) comprising:

   an ultrasonic probe (10) in which a plurality of elements for transmitting an ultrasonic wave to a subject and, by receiving an ultrasonic signal reflected from the subject, outputting the received signal are arrayed;
   a device (12, 14) which changes an assumed sonic speed set in advance relative to the actual sonic speed of the ultrasonic wave to be transmitted to the subject; and
   an optimum sonic speed judgment device (20) which judges a micro-structure by an RF signal obtained from the received signal by changing the assumed sonic speed and performing focusing with a delay based on the assumed sonic speed, and judges an optimum sonic speed, which is the ultrasonic speed of the subject, from phase information about the RF signal judged to be the micro-structure.

2. The ultrasonic diagnosis apparatus (1) according to claim 1, wherein the optimum sonic speed judgment device (20) judges the optimum sonic speed from amplitude information about the RF signal judged to be the micro-structure.

3. The ultrasonic diagnosis apparatus (1) according to claim 1, wherein the optimum sonic speed judgment device (20) judges the optimum sonic speed from phase change in the direction of the array of the elements of the ultrasonic probe, in the RF signal judged to be the micro-structure.

4. The ultrasonic diagnosis apparatus (1) according to claim 1, wherein the optimum sonic speed judgment device (20) judges the optimum sonic speed from phase change which depends on the assumed sonic speed, in the RF signal judged to be the micro-structure.

5. The ultrasonic diagnosis apparatus (1) according to claim 1, wherein the optimum sonic speed judgment device (20) judges the optimum sonic speed from amplitude change which depends on the assumed sonic speed, in the RF signal judged to be the micro-structure.

6. The ultrasonic diagnosis apparatus (1) according to any of claims 1 to 5, wherein the optimum sonic speed judgment device (20) uses a signal generated by changing a plurality of the assumed sonic speed from one transmission.

7. The ultrasonic diagnosis apparatus (1) according to any of claims 1 to 6, wherein the optimum sonic speed judgment device (20) uses a plurality of frames.

8. The ultrasonic diagnosis apparatus (1) according to claim 7, wherein the plurality of frames are obtained by a device which can generate RF data for two or more sound rays in scanning direction.

9. The ultrasonic diagnosis apparatus (1) according to any of claims 1 to 8, wherein the optimum sonic speed judgment device (20) uses such data that the resolution of the phase information is, in the direction of the array of the elements of the ultrasonic probe (10), equal to or more than the interval between the elements.

10. The ultrasonic diagnosis apparatus (1) according to any of claims 1 to 9, wherein the optimum sonic speed judgment device (20) obtains the optimum sonic speed for each of a plurality of the RF signal judged to be the micro-structures.

11. The ultrasonic diagnosis apparatus (1) according to any of claims 1 to 10, further comprising a display device (24) which displays one or more of images which have been generated under a plurality of optimum sonic speeds obtained for a plurality of the RF signal judged to be the micro-structures, respectively.

12. The ultrasonic diagnosis apparatus (1) according to claim 11, wherein the display device (24) displays an image obtained by synthesizing the images generated under the plurality of optimum sonic speeds.

13. The ultrasonic diagnosis apparatus (1) according to claim 11 or 12, further comprising a mode switching device (26) which switches the display mode of the display device (24) between a normal display mode and a display mode for displaying a plurality of images being overlapped or arranged or displaying one or more images.

14. An ultrasonic diagnosis apparatus (1) comprising:

an ultrasonic probe (10) in which a plurality of elements for transmitting an ultrasonic wave to a subject and, by receiving an ultrasonic signal reflected from the subject, outputting the received signal are arrayed;
a device (12, 14) which changes an assumed sonic speed set in advance relative to the actual sonic speed of the ultrasonic wave to be transmitted to the subject; and
an optimum sonic speed judgment device (20) which judges an optimum sonic speed, which is the ultrasonic speed of the subject, from phase change in the direction of the array of the elements, in an RF signal obtained from the received signal by, when the assumed sonic speed is changed, performing focusing with a delay based on the assumed sonic speed, in a predetermined target area.

15. The ultrasonic diagnosis apparatus (1) according to claim 14, wherein the optimum sonic speed judgment device (20) judges the optimum sonic speed from concave/convex change in the phase at a micro-structure, which depends on the assumed sonic speed.

16. The ultrasonic diagnosis apparatus (1) according to claim 14 or 15, wherein the optimum sonic speed judgment device (20) uses a signal generated by changing a plurality of the assumed sonic speed from one transmission.

17. The ultrasonic diagnosis apparatus (1) according to any of claims 14 to 16, wherein the optimum sonic speed judgment device (20) uses such data the resolution of the phase information is, in the direction of the array of the elements of the ultrasonic probe (10), equal to or more than the interval between the elements.

18. The ultrasonic diagnosis apparatus (1) according to any of claims 14 to 17, wherein the optimum sonic speed judgment device (20) obtains the optimum sonic speed for each of a plurality of target areas.

19. The ultrasonic diagnosis apparatus (1) according to any of claims 14 to 18, further comprising a display device (24) which displays one or more of images which have been generated under a plurality of optimum sonic speeds obtained for the plurality of target areas, respectively.

20. The ultrasonic diagnosis apparatus (1) according to claim 19, wherein the display device (24) displays an image obtained by synthesizing the images generated under the plurality of optimum sonic speeds.

21. The ultrasonic diagnosis apparatus (1) according to claim 19 or 20, further comprising a mode switching device (26) which switches the display mode of the display device (24) between a normal display mode and a display mode for displaying a plurality of images being overlapped or arranged or displaying one or more images.

# FIG.1

EP 2 044 886 A1

FIG.2

# FIG.3

EP 2 044 886 A1

Legend:
- SONIC SPEED 1500m/s
- SONIC SPEED 1540m/s
- SONIC SPEED 1580m/s
- SONIC SPEED 1620m/s

FIG.4

FIG.5

# FIG.6

EP 2 044 886 A1

# FIG.7

FIG.8

# FIG.9

EP 2 044 886 A1

# FIG.10

# FIG.11A    FIG.11B    FIG.11C

# FIG.12

```
                    START
                      |
                      v
          SET INITIAL VALUE
          OF ASSUMED SONIC SPEED    ~S100
                      |
                      v
          CHANGE ASSUMED
          SONIC SPEED BY 1 STEP     ~S110
                      |
                      v
          PERFORM PHASE
          ADJUSTMENT AND ADDITION    ~S120
          AND GENERATES RF DATA
                      |
                      v
    NO         HAS IMAGE             S130
  <------  GENERATION ENDED?
                      |
                    YES
                      |
                      v
                    END
```

FIG.13

```
                    ( START )
                        │
                        ▼
        ┌──────────────────────────────────┐
        │ SET INITIAL VALUE OF ASSUMED SONIC SPEED │─── S200
        └──────────────────────────────────┘
                        │
                        ▼
        ┌──────────────────────────────────┐
        │ INITIALIZE MICRO-STRUCTURE JUDGMENT │─── S202
        │ IMAGE & SONIC SPEED JUDGMENT IMAGE │
        └──────────────────────────────────┘
                        │
                        ▼
        ┌──────────────────────────────────┐
        │ CHANGE ASSUMED SONIC SPEED BY 1 STEP │─── S204
        └──────────────────────────────────┘
                        │
                        ▼
        ┌──────────────────────────────────┐
        │ CALCULATE SECONDARY DIFFERENTIAL │─── S206
        │ VALUE IN PHASE SCANNING DIRECTION │
        └──────────────────────────────────┘
                        │
                        ▼
        ┌──────────────────────────────────┐
        │ CALCULATE INTEGRATED VALUE AT │─── S208
        │ KERNEL WITH PREDETERMINED SIZE │
        └──────────────────────────────────┘
```

S214

STORE INTEGRATED VALUE IN SONIC SPEED JUDGMENT IMAGE FOR SPECIFIED ASSUMED SONIC SPEED

S210

TAKE ABSOLUTE VALUE

ADD ABSOLUTE VALUE TO MICRO-STRUCTURE JUDGMENT IMAGE

S212

S216

NO ← HAS PROCESSING ENDED FOR ALL ASSUMED SONIC SPEEDS?

YES

S218

RECORD POSITION WHERE VALUE IS EQUAL TO OR LARGER THAN PREDETERMINED VALUE IN MICRO-STRUCTURE JUDGMENT IMAGE, AS MICRO-STRUCTURE POSITION

JUDGE AND RECORD OPTIMUM SONIC SPEED, FOR EACH MICRO-STRUCTURE POSITION, FROM CHANGE IN VALUE IN SONIC SPEED JUDGMENT IMAGE FOR EACH ASSUMED SONIC SPEED

S220

( END )

## FIG.14

SONIC SPEED JUDGMENT IMAGE VALUE

1450m/s    OPTIMUM SONIC SPEED    1600m/s    ASSUMED SONIC SPEED

## FIG.15

SONIC SPEED JUDGMENT IMAGE VALUE USING AMPLITUDE

1450m/s    OPTIMUM SONIC SPEED    1600m/s    ASSUMED SONIC SPEED

## FIG.16

```
        ┌─────────────┐
        │    START    │
        └─────────────┘
               │
               ▼
┌──────────────────────────────────────┐
│ ACQUIRE EACH MICRO-STRUCTURE POSITION AND │ ─ S300
│ OPTIMUM SONIC SPEED VALUE AT EACH POSITION │
└──────────────────────────────────────┘
               │
               ▼
┌──────────────────────────────────────┐
│  ACQUIRE AMPLITUDE IMAGE CORRESPONDING │ ─ S310
│     TO EACH OPTIMUM SONIC SPEED        │
└──────────────────────────────────────┘
               │
               ▼
┌──────────────────────────────────────┐
│  SYNTHESIZE AMPLITUDE IMAGE FOR EACH   │
│  OPTIMUM SONIC SPEED, WITH EACH MICRO- │ ─ S320
│  STRUCTURE POSITION AS THE CENTER      │
└──────────────────────────────────────┘
               │
               ▼
┌──────────────────────────────────────┐
│       LOGARITHMIC COMPRESSION          │
│ GAIN, DR (DYNAMIC RANGE), STC (DEPTH   │ ─ S330
│ WEIGHTING) AND GRAY MAP ADJUSTMENTS    │
│            SCAN CONVERSION             │
└──────────────────────────────────────┘
               │
               ▼
        ┌─────────────┐
        │     END     │
        └─────────────┘
```

## FIG.17A

FASTER THAN OPTIMUM
SONIC SPEED

30

## FIG.17B

SLOWER THAN OPTIMUM
SONIC SPEED

32

FIG.18A  ASSUMED SONIC SPEED < OPTIMUM SONIC SPEED

(1) ISOLATED POINT

PHASE CONCAVE/CONVEX VALUE

(2) COMPLETE SPECKLE

PHASE CONCAVE/CONVEX VALUE

(3) SPECKLE INCLUDING
    LOCALLY HIGH ECHO

PHASE CONCAVE/CONVEX VALUE

FIG.18B  ASSUMED SONIC SPEED > OPTIMUM SONIC SPEED

(1) ISOLATED POINT

PHASE CONCAVE/CONVEX VALUE

(2) COMPLETE SPECKLE

PHASE CONCAVE/CONVEX VALUE

(3) SPECKLE INCLUDING
    LOCALLY HIGH ECHO

PHASE CONCAVE/CONVEX VALUE

# FIG.19

```
        ┌─────────────────────┐
        │        START        │
        └─────────────────────┘
                  │
                  ▼
  ┌───────────────────────────────────┐
  │              SET ROI              │──── S400
  └───────────────────────────────────┘
                  │
                  ▼
  ┌───────────────────────────────────┐
  │ SET INITIAL VALUE OF ASSUMED SONIC SPEED │──── S410
  └───────────────────────────────────┘
                  │
                  ▼
  ┌───────────────────────────────────┐
  │  CHANGE ASSUMED SONIC SPEED BY 1 STEP  │──── S420
  └───────────────────────────────────┘
                  │
                  ▼
  ┌───────────────────────────────────┐
  │  QUANTIFY PHASE CONCAVE/CONVEX IN      │
  │  SCANNING DIRECTION WITHIN ROI         │──── S430
  └───────────────────────────────────┘
                  │
                  ▼
        NO ◇ HAS PROCESSING           ◇── S440
           ◇ ENDED FOR ALL ASSUMED SONIC ◇
           ◇        SPEEDS?              ◇
                  │ YES
                  ▼
  ┌───────────────────────────────────┐
  │ JUDGE OPTIMUM SONIC SPEED FROM CHANGE IN │
  │ UNEVEN PHASE CONCAVE/CONVEX DISTRIBUTION │──── S450
  │ WITHIN ROT UNDER EACH ASSUMED SONIC SPEED │
  └───────────────────────────────────┘
                  │
                  ▼
        ┌─────────────────────┐
        │         END         │
        └─────────────────────┘
```

# FIG.20

# FIG.21

```
                    ┌──────────────┐
                    │    START     │
                    └──────┬───────┘
                           │
                           ▼
  ┌─────────────────────────────────────────────┐
  │      ACQUIRE OPTIMUM SONIC SPEED             │
  │         VALUE IN EACH ROI                    │──── S500
  └─────────────────────┬───────────────────────┘
                        │
                        ▼
  ┌─────────────────────────────────────────────┐
  │   ACQUIRE AMPLITUDE IMAGE CORRESPONDING      │
  │      TO EACH OPTIMUM SONIC SPEED             │──── S510
  └─────────────────────┬───────────────────────┘
                        │
                        ▼
  ┌─────────────────────────────────────────────┐
  │    SYNTHESIZE AMPLITUDE IMAGE FOR EACH       │
  │    OPTIMUM SONIC SPEED, WITH EACH ROI        │──── S520
  │         POSITION AS THE CENTER               │
  └─────────────────────┬───────────────────────┘
                        │
                        ▼
  ┌─────────────────────────────────────────────┐
  │         LOGARITHMIC COMPRESSION              │
  │   GAIN, DR (DYNAMIC RANGE), STC (DEPTH       │──── S530
  │   WEIGHTING) AND GRAY MAP ADJUSTMENTS        │
  │            SCAN CONVERSION                   │
  └─────────────────────┬───────────────────────┘
                        │
                        ▼
                 ┌──────────────┐
                 │     END      │
                 └──────────────┘
```

**EUROPEAN SEARCH REPORT**

Europäisches
Patentamt
European
Patent Office
Office européen
des brevets

Application Number

EP 08 01 7303

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2007/083110 A1 (LIN SHENGTZ [US] ET AL) 12 April 2007 (2007-04-12) * abstract * * paragraphs [0001], [0025] - [0027], [0032] - [0038], [0041] * * figures 3,4A,4B,5 * | 1-21 | INV. A61B8/00 G10K11/34 G01N29/44 G01H5/00 G01S7/52 |
| A | FERNANDEZ A T ET AL: "High resolution ultrasound beamforming using synthetic and adaptive imaging techniques" BIOMEDICAL IMAGING, 2002. PROCEEDINGS. 2002 IEEE INTERNATIONAL SYMPOSIUM ON JULY 7-10, 2002, PISCATAWAY, NJ, USA, IEEE, 7 July 2002 (2002-07-07), pages 433-436, XP010600618 ISBN: 978-0-7803-7584-0 Section "3. METHODS AND RESULTS - ABERRATION MEASUREMENTS" | 1,14 | |
| A | US 4 627 290 A (OGAWA TOSHIO [JP] ET AL) 9 December 1986 (1986-12-09) * abstract * | | TECHNICAL FIELDS SEARCHED (IPC) A61B G01S G10K G01N |
| A | WO 2007/075040 A (MEDISON CO LTD [KR]; JEONG MOK KEUN [KR]; KWON SUNG JAE [KR]; YOON RA) 5 July 2007 (2007-07-05) * abstract * | | |
| A | EP 0 119 019 A (HITACHI LTD [JP]) 19 September 1984 (1984-09-19) * abstract * | | |
| A | EP 1 011 093 A (MEDISON CO LTD [KR]) 21 June 2000 (2000-06-21) * abstract * | | |

-/--

The present search report has been drawn up for all claims

| Place of search | Date of completion of the search | Examiner |
|---|---|---|
| Munich | 4 February 2009 | Willig, Hendrik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches
Patentamt

European
Patent Office

Office européen
des brevets

**EUROPEAN SEARCH REPORT**

**Application Number**

EP 08 01 7303

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 1 262 148 A (HITACHI MEDICAL CORP [JP]) 4 December 2002 (2002-12-04) * abstract * ----- | | |
| A | US 5 638 820 A (CHEN JIAN-FENG [US] ET AL) 17 June 1997 (1997-06-17) * abstract * ----- | | |

**TECHNICAL FIELDS SEARCHED (IPC)**

The present search report has been drawn up for all claims

| Place of search | Date of completion of the search | Examiner |
|---|---|---|
| Munich | 4 February 2009 | Willig, Hendrik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.** EP 08 01 7303

This annex lists the patent family members relating to the patent documents cited in the above-mentioned European search report.
The members are as contained in the European Patent Office EDP file on
The European Patent Office is in no way liable for these particulars which are merely given for the purpose of information.

04-02-2009

| Patent document cited in search report | | Publication date | Patent family member(s) | | Publication date |
|---|---|---|---|---|---|
| US 2007083110 | A1 | 12-04-2007 | NONE | | |
| US 4627290 | A | 09-12-1986 | JP | 1885990 C | 22-11-1994 |
| | | | JP | 6009561 B | 09-02-1994 |
| | | | JP | 60220051 A | 02-11-1985 |
| WO 2007075040 | A | 05-07-2007 | EP | 1965704 A1 | 10-09-2008 |
| | | | KR | 20070069425 A | 03-07-2007 |
| | | | US | 2009003128 A1 | 01-01-2009 |
| EP 0119019 | A | 19-09-1984 | DE | 3474862 D1 | 01-12-1988 |
| | | | JP | 1882623 C | 10-11-1994 |
| | | | JP | 6004074 B | 19-01-1994 |
| | | | JP | 59149132 A | 27-08-1984 |
| | | | US | 4566459 A | 28-01-1986 |
| EP 1011093 | A | 21-06-2000 | JP | 3397734 B2 | 21-04-2003 |
| | | | JP | 2000166925 A | 20-06-2000 |
| | | | KR | 20000038847 A | 05-07-2000 |
| | | | US | 6305225 B1 | 23-10-2001 |
| EP 1262148 | A | 04-12-2002 | WO | 0166014 A1 | 13-09-2001 |
| | | | JP | 2001252276 A | 18-09-2001 |
| | | | US | 2003092990 A1 | 15-05-2003 |
| US 5638820 | A | 17-06-1997 | NONE | | |

EPO FORM P0459

For more details about this annex : see Official Journal of the European Patent Office, No. 12/82

**EP 2 044 886 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 8317926 A